# EUROPÄISCHE PATENTSCHRIFT

(11) **EP 3 273 936 B1**
(45) Veröffentlichungstag und Bekanntmachung des Hinweises auf die Patenterteilung: **08.05.2019**
(21) Anmeldenummer: 16713758.7
(22) Anmeldetag: 16.03.2016
(51) Int. Cl.: A61K 8/49, A61Q 5/02

(54) **PERLGLANZKONZENTRATE IN WÄSSRIGEN, TENSIDISCHEN MITTELN**
PEARLY LUSTER CONCENTRATES IN AQUEOUS MEDIA CONTAINING SURFACTANTS
CONCENTRÉS NACRÉS DANS DES MILIEUX TENSIOACTIFS AQUEUX

(30) Priorität: 26.03.2015 EP 15161017
(43) Veröffentlichungstag der Anmeldung: 31.01.2018
(73) Patentinhaber: BASF SE, 67056 Ludwigshafen am Rhein (DE)
(72) Erfinder: NIEENDICK, Claus, 47807 Krefeld (DE); OHLMANN, Dominik, 68163 Mannheim (DE); VAN DEN WITTENBOER, Anne, 40239 Düsseldorf (DE)
(74) Vertreter: BASF IP Association
(86) Internationale Anmeldenummer: PCT/EP2016/055650
(87) Internationale Veröffentlichungsnummer: WO 2016/150786

(56) Entgegenhaltungen:
- EP-A1- 1 067 175
- DE-A1- 19 810 888
- US-A- 3 014 927
- US-B1- 6 235 702

## Beschreibung

### Gebiet der Erfindung

Die Erfindung liegt auf dem Gebiet von wässrigen, tensidischen Mitteln mit Perlglanzeffekt und betrifft die Verwendung spezieller Ester des THF-Glykols als Perlglanzwachs sowie Perlglanzkonzentrate enthaltend Ester des THF-Glykols als auch insbesondere kosmetische Mittel enthaltend die Perlglanzwachse bzw. die Ester des THF-Glykols.

### Stand der Technik

Der weich schimmernde Glanz von Perlen hat auf den Menschen schon seit Jahrtausenden eine besondere Faszination ausgeübt. Es ist daher kein Wunder, dass die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Für die moderne Kosmetik sind hingegen Perlglanzwachse, insbesondere vom Typ der Glykolmono- und -difettsäureester von Bedeutung, die überwiegend zur Erzeugung von Perlglanz in Haarshampoos und Duschgelen eingesetzt werden.

Der Stand der Technik kennt eine Vielzahl von Formulierungen, die oberflächenaktiven Reinigungsmitteln den gewünschten Perlglanz verleihen. So lassen sich beispielsweise gemäß der Lehre der Europäischen Patentschrift EP-B1 0 569 843 (Hoechst) nichtionische, fließfähige Perlglanzdispersionen erhalten, indem man Mischungen aus 5 bis 30 Gew.-% acylierten Polyglycolen und 0,1 bis 20 Gew.-% ausgewählten nichtionischen Tensiden herstellt. Aus der Europäischen Patentanmeldung EP-A2 0 581 193 (Hoechst) sind ferner fließfähige, konservierungsmittelfreie Perlglanzdispersionen bekannt, die acylierte Polyglycolether, Betaine, Aniontenside und Glycerin enthalten.

Trotz der Vielzahl von Mitteln besteht im Markt ein ständiges Bedürfnis nach neuen Perlglanzwachsen, insbesondere an Perlglanzwachsen, die möglichst keine Ethylenoxid und/oder Propylenoxid-Einheiten aufweisen, da diese breit einsetzbar sind, sowohl in klassischen als auch in sogenannten "grünen" Reinigungsmitteln mit Tensiden ohne Ethylenoxideinheiten. Dennoch wird von den neuen Perlglanzwachsen erwartet, dass sie ein vergleichbares Leistungsspektrum hinsichtlich Perlglanz, insbesondere Weißgrad und brillanten Glanz, aufweisen wie die weit verbreiteten Polyethylenglykolstearate. Zudem sollen die Perlglanzwachse auch mit den anderen, sensiblen bzw. kritischen Inhaltsstoffen wie Siliconen eine ausreichende Verträglichkeit aufweisen, so dass die Stabilität der Reinigungsmittel nicht beeinträchtigt wird. Des Weiteren wird erwartet, dass man die Perlglanzmittel leicht, d.h. ohne großen technischen und energetischen Aufwand in die kosmetische Formulierung einarbeiten kann. Dies soll möglichst einfach durch Rühren bei Raumtemperatur, ohne zeit- und kostenintensive Erwärmung der kosmetischen Formulierung erfolgen. Zusätzlich besteht bei vielen Herstellern der kosmetischen Mittel ein Bedürfnis nach Perlglanzwachsen, die als Konzentrat formulierbar sind, um unnötige Transport- und Lagerkosten zu vermeiden. Diese Konzentrate sollen zusätzlich fließfähig sein, um eine leichte Herstellbarkeit der kosmetischen Formulierung zu gewährleisten.

Gewünscht werden im Zuge der Nachhaltigkeit zudem Verbindungen, die ökologisch nachhaltig und ausreichend gut biologisch abbaubar sind.

Die Aufgabe der vorliegenden Erfindung hat somit darin bestanden, neue Perlglanzwachse bereitzustellen, die dem geschilderten komplexen Anforderungsprofil gerecht werden.

Die Aufgabe konnte gelöst werden mittels ausgewählter Ester des 2,5-Di(hydroxymethyl)tetrahydrofurans (= THF-Glykol).

2,5-Di(hydroxymethyl)tetrahydrofuran (IUPAC: [5-(Hydroxymethyl)oxolan-2-yl]methanol, im Folgenden auch THF-Glykol genannt) ist kommerziell erhältlich und wird beispielsweise zur Herstellung von Weichmachern, Polymerharzen und als Lösungsmittel verwendet.

Kurzkettige Mono- und Diester (A) des 2,5-Di(hydroxymethyl)tetrahydrofurans 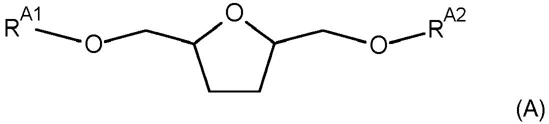 Monoester: R^{A1} = Acyl, R^{A2} = H, Diester: R^{A1} = Acyl, R^{A2} = Acyl
sind bekannt aus Jung et al. in Heterocycles 1993, 35, 273-280, wobei das Diol mit Mosher-Säure (α-Methoxy α-trifluormethylphenylessigsäure) in Gegenwart von Dicyclohexylcarbodiimid und 4-Dimethylaminopyridin umgesetzt wird.

Aus der Amerikanischen Patentschrift US 3014927 ist bekannt, dass Monoester von 2,5 Tetrahydrofurandi-methanol durch Umsetzung von 5-Hydroxymethylfurfural mit Säureanhydrid oder Säurechlorid in Anwesenheit eines Katalysators wie Pyridin und nachfolgender Hydrierung zugänglich sind.

In der noch unveröffentlichten Europäischen Patentanmeldung EP14187932.0 vom 07.10.2014 der Anmelderin wird die chemische und enzymatische Herstellung von kurz- und langkettigen Mono- und Diester des THF-Glykols beschrieben sowie deren Eignung als Tensid und als Solubilisator in kosmetischen Mitteln.

### Zusammenfassung der Erfindung

Gegenstand der vorliegenden Anmeldung ist die Verwendung von Estern des 2,5-Di(hydroxymethyl)tetrahydrofurans der allgemeinen Formel (I), 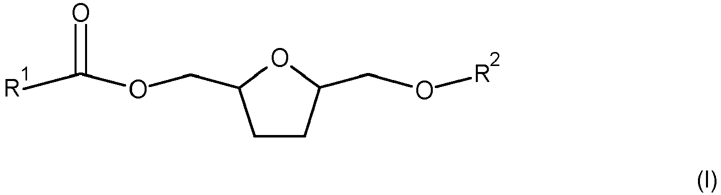 worin
R² für C(=O)R^{1'} steht und wobei R¹ und R^{1'} unabhängig voneinander für einen C13-C25-Alkylrest steht, der ggf. mit einer Hydroxylgruppe substituiert ist
   oder
R² für Wasserstoff steht und R¹ für einen C13-C25-Alkylrest, der ggf. mit einer Hydroxylgruppe substituiert ist, oder Mischungen derartiger Ester
   als Perlglanzwachs in wässrigen, tensidischen Zubereitungen.

Ein weiterer Gegenstand der vorliegenden Erfindung betrifft Perlglanzkonzentrate für wässrige, tensidische Zubereitungen enthaltend
(a) 7,5 bis 35 Gew.-% Ester des 2,5-Di(hydroxymethyl)tetrahydrofurans der allgemeinen Formel (I) gemäß Anspruch 1
(b) 0,1 bis 60 Gew-% anionische, nichtionische, kationische, ampholytische oder zwitterionische Tenside oder deren Kombinationen
(c) 0 - 40 Gew.-% Polyole
(d) ad 100 Gew.-% Wasser.

Weitere Gegenstände der vorliegenden Erfindung betreffen ein Verfahren zur Herstellung von Perlglanzkonzentraten, wobei man die Ester des 2,5-Di(hydroxymethyl)tetrahydrofurans der allgemeinen Formel (I) gemäß Anspruch 1 sowie die Tenside (b) sowie ggf. die Polyole (c) und ad 100 Gew.-% Wasser unter Rühren auf Temperaturen erwärmt, die 5 bis 20 °C über dem Schmelzpunkt des Esters der Formel (I) liegen, rührt und anschließend die Mischung unter stetigem Rühren auf etwa Raumtemperatur (20 bis 23 °C) abkühlt oder wobei man zu einer wässrigen Paste der Tenside (b) die Ester des 2,5-Di(hydroxymethyl)tetrahydrofurans der allgemeinen Formel (I) einrührt, auf Temperaturen, die 5 bis 20 °C über dem Schmelzpunkt des Esters liegen, erwärmt, rührt und anschließend mit weiterem Wasser und ggf. Polyolen die gewünschte Konzentration einstellt.

Die Verwendung der Perlglanzkonzentrate zur Herstellung von wässrigen, tensidischen Zubereitungen mit Perlglanzeffekt sowie wässrige, tensidische Zubereitungen mit Perlglanzeffekt enthaltend 0,2 bis 10 Gew.-% Perlglanzkonzentrat sind weitere Gegenstände der Erfindung. Schließlich betrifft die Erfindung noch wässrige, tensidische Zubereitungen enthaltend 0,015 bis 3,5 Gew.-% Ester des 2,5-Di(hydroxymethyl)tetrahydrofurans der allgemeinen Formel (I).

Die verwendeten Mono- und Diester des THF-Glykols zeigen als Perlglanzwachs in vorteilhafter Weise:
- einen ausgeprägten Perlglanzeffekt, der vergleichbar ist mit dem besten Standard
- sehr gute Werte in der Brillanz
- sehr guter Weißgrad
- leichte Überführung in Konzentrate, die zudem fließfähig sind
- formulierbar als stabile Konzentrate,
- Perlglanzwachse können als Konzentrate leicht in kosmetische, tensidische Zubereitungen eingearbeitet werden
- die erhaltenen kosmetischen Mittel sind lagerstabil

### Beschreibung der vorliegenden Erfindung

Sofern im Folgenden nicht anders angegeben, werden die Begriffe Ester des "2,5-Di(hydroxymethyl)tetrahydrofurans", Ester des "Tetrahydrofuranglykols" auch verkürzt als "THF-Glykol"-ester bzw. "Ester des THF-Glykols" bezeichnet.

Im Rahmen der Erfindung umfassen diese Begriffe auch cis/trans-Gemische jedweder Zusammensetzung sowie die reinen Konfigurations-Isomere. Die zuvor genannten Begriffe bezeichnen weiterhin alle Enantiomere in Reinform sowie racemische und optisch aktive Gemische der Enantiomeren dieser Verbindungen.

Lediglich zur Veranschaulichung werden im Folgenden die Isomeren eines Monoesters des THF-Glykols wiedergegeben:

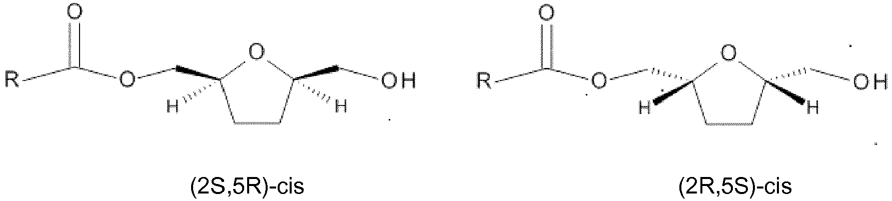

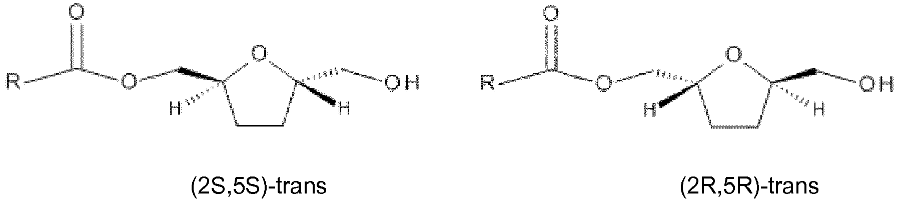

Im Sinne der Erfindung wird der Begriff "Perlglanzwachs" für wachsartige Verbindungen verwendet, die einen Perlglanzeffekt bewirken (anstelle des Begriffes "Perlglanz" sind auch die Begriffe "Perlmuttglanz" oder "Lüster" gebräuchlich). Der Perlglanzeffekt wird im Sinne der Erfindung als Weißgrad und Brillanz, die Intensität des Schimmers, optisch im Vergleich zu dem Standard Ethylenglykoldistearat als Perlglanzwachs nach dem Schulnotensystem beurteilt, wobei der Standard die Bestnote 1 erhält.

Eine wachsartige Verbindung ist im Sinne der Erfindung eine bei Raumtemperatur feste, meist knetbare Verbindung, die ohne Zersetzung meist über 30 °C, vorzugsweise über 40 °C, und insbesondere zwischen 40 und 65 °C, gemessen gemäß ISO 6321.

Im Rahmen der Erfindung wird der Begriff der "tensidischen Zubereitungen" verstanden als Mittel, enthaltend tensidische Verbindungen. Im Sinne der vorliegenden Erfindung werden unter "tensidische Verbindungen" Tenside, Emulgatoren als auch tensidische, polymere Verbindungen verstanden. "Tenside" bilden aufgrund ihrer Struktur an der Wasseroberfläche eine dünne Schicht und senken damit die Oberflächenspannung des Wassers. Verbindungen, die bewirken, dass zwei nicht-mischbare Flüssigkeiten eine Emulsion bilden, werden häufig auch als Emulgatoren bezeichnet, die im Sinne der Erfindung unter dem Begriff "Tenside" subsumiert werden.

Im Sinne der Erfindung werden Ester der Formel (I) verwendet, 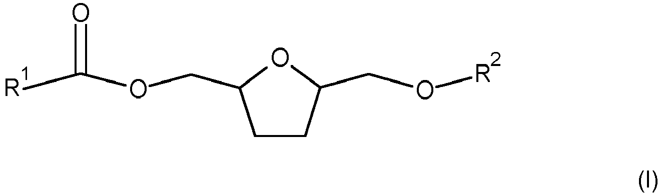 worin
R² für C(=O)R^{1'} steht und wobei R¹ und R^{1'} unabhängig voneinander für einen C13-C25-Alkylrest steht, der ggf. mindestens mit einer Hydroxylgruppe substituiert ist
   oder
R² für Wasserstoff steht und R¹ für einen C13-C25-Alkylrest, der ggf. mindestens mit einer Hydroxylgruppe substituiert ist, oder Mischungen derartiger Ester
   als Perlglanzwachs in wässrigen, tensidischen Zubereitungen.

Die Verbindungen der Formel (I) und deren Herstellung werden in der noch unveröffentlichten Europäischen Patentanmeldung **P**14187932.0 vom 07.10.2014 beschrieben.

Demnach können die Verbindungen der Formel (I) chemisch hergestellt werden durch Veresterung von 2,5-Di(hydroxymethyl)tetrahydrofuran mit wenigstens einem Säurehalogenid R¹-C(=O)X und, falls R² für -(C=O)R^{1'} steht und R¹ und R^{1'} unterschiedliche Bedeutung haben, zusätzlich mit wenigstens einem Säurehalogenid R^{1'}-C(=O)X, wobei X für Br oder Cl steht, in Gegenwart wenigstens eines tertiären Amins.

Die Ausgangsverbindung 2,5-Di(hydroxymethyl)tetrahydrofuran ist beispielsweise durch Hydrierung von 2,5-Di(hydroxymethyl)furan erhältlich. 2,5-Di(hydroxymethyl)furan kann z. B. ausgehend von Fructose durch Dehydrierung zu 5-Hydroxymethylfurfural und anschließende Reduktion der Formylgruppe hergestellt werden. Somit ist eine Herstellung des 2,5-Di(hydroxymethyl)tetrahydrofurans aus biogenen Quellen, ausgehend von entsprechenden Kohlehydraten, z. B. Stärke, Cellulose und Zuckern, möglich.

Zur Veresterung können üblicherweise alle dem Fachmann geläufige Arten von tertiären Aminen verwendet werden. Beispiele für geeignete tertiäre Amine sind:
- aus der Gruppe der Trialkylamine: Trimethylamin, Triethylamin, Tri-n-propylamin, Diethylisopropylamin, Diisopropylethylamin und dergleichen;
- aus der Gruppe der N-Cycloalkyl-N,N-dialkylamine: Dimethylcyclohexylamin und Diethylcyclohexylamin;
- aus der Gruppe der N,N-Dialkylaniline: Dimethylanilin und Diethylanilin;
- aus der Gruppe der Pyridin- und Chinolinbasen: Pyridin, α-, β- und γ-Picolin, Chinolin und 4-(Dimethylamino)pyridin (DMAP).

Bevorzugte tertiäre Amine sind Trialkylamine und Pyridinbasen, insbesondere Triethylamin und 4-(Dimethylamino)pyridin (DMAP) sowie Gemische davon.

In einer bevorzugten Ausführungsform wird zur Veresterung ein Trialkylamin, vorzugsweise ausgewählt unter Trimethylamin, Triethylamin, Tri-n-propylamin, Diethylisopropylamin und Diisopropylethylamin eingesetzt. Das Trialkylamin wird dabei bevorzugt in einem wenigsten stöchiometrischen Verhältnis, bezogen auf die Hydroxylgruppen des 2,5-Di-(hydroxymethyl)tetrahydrofurans eingesetzt. Besonders bevorzugt wird das Trialkylamin in einem wenigstens stöchiometrisches Verhältnis bis zu einem vierfachen stöchiometrischen Überschuss, bezogen auf die Hydroxylgruppen des 2,5-Di-(hydroxymethyl)tetrahydrofurans eingesetzt.

Bevorzugt wird zur Veresterung zusätzlich zu wenigstens einem Trialkylamin noch Dimethylaminopyridin eingesetzt. Es wird angenommen, dass Dimethylaminopyridin dabei als Katalysator wirkt. Die Einsatzmenge des Dimethylaminopyridins liegt vorzugsweise in einem Bereich von 0,01 bis 0,5 Moläquivalenten, besonders bevorzugt 0,05 bis 0,2 Moläquivalenten, bezogen auf 2,5-Di-(hydroxymethyl)tetrahydrofuran.

Bevorzugt wird die Veresterung in einem Temperaturbereich von -10 bis 75 °C, bevorzugt 0 bis 60 °C, durchgeführt. Bei diesen niedrigen Temperaturen wird eine thermische Zersetzung des 2,5-Di-(hydroxymethyl)tetrahydrofurans vermieden, wie sie bei den aus dem Stand der Technik bekannten Verfahren im stark sauren Medium und bei erhöhter Temperatur beobachtet wird. Des Weiteren wird bei diesen niedrigen Temperaturen eine gute Selektivität bezüglich des jeweiligen Verfahrensprodukts (Monoester oder Diester) erzielt.

Die Veresterung kann bei Umgebungsdruck, bei vermindertem oder erhöhtem Druck erfolgen. Bevorzugt wird die Veresterung bei Umgebungsdruck durchgeführt.

Die Veresterung kann in Abwesenheit oder in Gegenwart eines organischen Lösungsmittels durchgeführt werden. Bevorzugt wird die Reaktion in Gegenwart eines inerten organischen Lösungsmittels durchgeführt, vorzugsweise ausgewählt unter aliphatischen Ethern, cyclischen Ethern, Ketonen, chlorierten Kohlenwasserstoffen, aliphatischen Kohlenwasserstoffen, cycloaliphatischen Kohlenwasserstoffen, Aromaten und Gemischen davon. Geeignete Lösungsmittel sind THF, Dioxan, Methyl-tert-butylether, Diethylether, Aceton, Methylethylketon, Dichlormethan, Trichlormethan, Tetrachlormethan, Pentan, Hexan, Heptan, Ligroin, Petrolether, Cyclohexan, Benzol, Toluol, Xylol, Chlorbenzol, Dichlorbenzole und Mischungen davon. Bevorzugt wird die Reaktion in Gegenwart eines im Wesentlichen wasserfreien organischen Lösungsmittels durchgeführt.

Die Alkylierung kann in Abwesenheit oder in Gegenwart eines unter den Reaktionsbedingungen inerten Gases, wie Stickstoff oder Argon, erfolgen. Bevorzugt wird bei der Veresterung kein Inertgas eingesetzt.

Das Verhältnis der entstehenden Monoester (R² = H) zu den Diestern (R² steht für C(=O)R^{1'}) kann über die Stöchiometrie der Rektanden gesteuert werden.

Zur Herstellung von Diester wird das Säurehalogenid R¹-(C=O)X oder die Gesamtmenge der Säurehalogenide R¹-(C=O)X und R^{1'}-(C=O)X in wenigstens äquimolarer Menge oder einem molaren Überschuss, bezogen auf die Hydroxylgruppen des 2,5-Di-(hydroxymethyl)tetrahydrofurans eingesetzt. Für die Herstellung von Monoester-haltigen Gemischen wird das Säurehalogenid R¹-(C=O)X oder die Gesamtmenge der Säurehalogenide R¹-(C=O)X und R^{1'}-(C=O)X in einem entsprechenden Unterschuss, bezogen auf die Hydroxylgruppen des 2,5-Di-(hydroxymethyl)tetrahydrofurans eingesetzt.

Im Sinne der vorliegenden Erfindung sind Verbindungen der Formel (I) bevorzugt, die durch Umsetzung von 2,5-Di(hydroxymethyl)tetrahydrofuran mit wenigstens einer Verbindung R¹-COOH und, falls R² für C(=O)R^{1'} steht und R¹ und R^{1'} unterschiedliche Bedeutung haben, zusätzlich mit einer davon verschiedenen Verbindung R^{1'}-COOH in Gegenwart eines enzymatischen Veresterungskatalysators hergestellt werden.

Bevorzugt enthält der enzymatische Veresterungskatalysator wenigstens ein Enzym, das ausgewählt ist unter Hydrolasen, bevorzugt Lipasen, oder Esterasen. Besonders bevorzugt ist das Enzym ausgewählt unter Lipasen aus Candida rugosa, Candida antarctica, Thermomyces lanunginosa, Rhizomucor miehei oder Esterase aus Schweineleber. Insbesondere bevorzugt sind Lipasen, speziell Lipasen aus Candida antarctica, ganz speziell Lipase B aus Candida antarctica.

Der enzymatische Veresterungskatalysator kann als Zellextrakt, gereinigte Proteinlösung (homogener Katalysator) oder in immobilisierter (auf einem Träger gebundener) Form (heterogener Katalysator) eingesetzt werden. Bevorzugt wird das Enzym in immobilisierter Form eingesetzt.

Zur Immobilisierung werden die Enzyme bevorzugt entweder adsorptiv, ionisch oder kovalent an anorganische oder organische Trägerpartikel gebunden. Dies gehört zum Wissen des einschlägigen Fachmanns.

Als organische Träger können insbesondere solche eingesetzt werden, die Polyacrylat, Polymethacrylat, Polyvinylstyrol, Styrol-Divinylbenzolcopolymeren, Polypropylen, Polyethylen, Polyethylentherephthalat, Polytetrafluorethylen (PTFE) und/oder andere Polymere aufweisen oder aus diesen bestehen. Als Trägermaterial können, in Abhängigkeit von dem zu immobilisierenden Enzym, auch saure oder basische lonenaustauscherharze eingesetzt werden, beispielsweise Duolite® A568, Duolite® XAD 761, Duolite® XAD 1180, Duolite® XAD 7HP, Amberlite® IR 120, Amberlite® IR 400, Amberlite® CG 50, Amberlyst® 15 (alles Produkte der Fa. Rohm und Haas) und Lewatit® CNP 105 (Produkte der Fa. Lanxess, Leverkusen, Deutschland). Bevorzugte Träger sind Polypropylenträger oder Acrylatträger. Insbesondere bevorzugt sind der Polypropylenträger Accurel® MP1000 oder der Acrylatträger Lewatit® VP OC 1600. Ein bevorzugtes kommerziell erhältliches Enzym ist ein Präparat der Lipase B aus Candida antarctica (CAL-B) (Novozyme® 435) immobilisiert auf einem Polymethacrylatträger.

Als anorganische Träger können aus dem Stand der Technik bekannte, oxidische und/oder keramische Träger eingesetzt werden. Insbesondere können als anorganische Träger Celite, Zeolite, Silica, Controlled-Pore Glas (CPG) oder andere Träger eingesetzt werden.

Vorzugsweise weist der eingesetzte Träger eine Teilchengrößenverteilung auf, bei der mindestens 90 % der Teilchen eine Teilchengröße von 0,5 bis 5000 µm, bevorzugt von 1 bis 2000 µm, besonders bevorzugt von 10 bis 2000 µm, insbesondere 25 bis 2000 µm aufweisen.

Das hier beschriebene Verfahren zur enzymatischen Veresterung erfolgt bevorzugt in einem Reaktor, der wenigstens einen heterogenen Katalysator enthält. Der heterogene Katalysator liegt vorzugsweise in Form eines Festbetts vor oder befindet sich suspendiert im Reaktionsgemisch.

Vorteilhaft am Festbett ist, dass das geträgerte Enzym nicht extra vom Produkt abgetrennt werden muss und die Trägerpartikel nicht in Kontakt mit einem Rührwerk kommen können. Die Partikel bleiben so besser unversehrt. Nachteilig ist allerdings, dass die Reaktanden meist mehrmals über ein solches Festbett geleitet werden müssen, um einen annehmbaren Reaktionsumsatz zu erzielen. Zudem kann sich die Entfernung von Reaktionswasser schwieriger gestalten, ein Druckverlust über das Festbett resultieren oder Entmischungsphänomene auftreten.

Im Suspensionsreaktor wird das Reaktionsgemisch üblicherweise mithilfe einer Rührvorrichtung durchmischt. Sollte dies der Fall sein, dann unterliegen die Trägerpartikel einer gewissen mechanischen Belastung. Es gibt allerdings Möglichkeiten, über die Wahl der Reaktorgeometrie auf eine Rührvorrichtung zu verzichten und die nötige Durchmischung anderweitig zu gewährleisten (z. B. durch Einsatz einer Blasensäule oder eines Airlift-Reaktors). Vorteilhaft am Suspensionsreaktor ist ein Reaktionsumsatz in einem Schritt. Dabei ist vorteilhafterweise bei Verwendung eines heterogenen Katalysators die Abtrennung des Reaktionsprodukts durch Größenausschlussverfahren möglich, z. B. unter Verwendung eines Siebes, mit dessen Hilfe Feststoffe nach dem Kriterium der Korngröße abgetrennt werden können. Im Speziellen kann es sich bei dem Siebverfahren auch um eine Filtration handeln. Die Größe und/oder Geometrie der Öffnungen des Trennmediums (beispielsweise Filterporen) orientiert sich dabei an der kleinsten abzutrennenden Korngröße. Zudem kann eine derartige Abtrennung auch sequenziell durch Hintereinanderschalten mehrerer unterschiedlicher Siebe und/oder Filter (unterschiedlich z. B. in der Größe und/oder Geometrie der Öffnungen des Trennmediums) erfolgen. Die Auswahl orientiert sich häufig daran, dass eine Abtrennung möglichst ohne Druckverlust erfolgt.

Vorteilhaft am Getrennthalten des geträgerten Enzyms von der Reaktionsmischung (z. B. mittels Festbett) oder der Abtrennbarkeit des geträgerten Enzyms von Suspensionen ist, dass die Enzyme auf diese Art mehrmals verwendet werden können. Die Wiederverwendung des Enzyms in Form der geträgerten Variante (als heterogener Katalysator) ist auch deswegen bevorzugt.

Bevorzugt wird die enzymatische Veresterung ohne Zusatz eines externen Lösungsmittels (hier "lösungsmittelfrei" genannt) durchgeführt.

Bei der Estersynthese entsteht Reaktionswasser, welches zu einer unerwünschten Verschiebung des Reaktionsgleichgewichts führt. Vorzugsweise wird das bei der Reaktion gebildete Wasser nach üblichen, dem Fachmann bekannten Verfahren entfernt. Der Menge des ausgetragenen Reaktionswassers wird so gewählt, dass das Reaktionsgleichgewicht ausreichend in Bezug auf das angestrebte Produkt (Ester) verschoben wird. Der Umsatz der Edukte (Alkohol und Säure) soll mit anderen Worten möglichst vollständig sein, um die maximal mögliche Esterausbeute zu erzielen. Der angestrebte Umsatz liegt im Allgemeinen bei größer 80 %, vorzugsweise größer 85 %, besonders bevorzugt größer 90 %, insbesondere größer 92 %, insbesondere größer 94 %, insbesondere größer 96 %, insbesondere größer 98 %, insbesondere größer 99 %, insbesondere größer 99,2 %, insbesondere größer 99,4 %, insbesondere größer 99,6 %, insbesondere größer 99,7 %, insbesondere größer 99,8 %.

Die Entfernung des Reaktionswassers kann durch folgende Maßnahmen erfolgen:
- destillative Entfernung des Wassers,
- Einsatz eines Strip- oder Schleppgases,
- Einsatz wenigstens eines Trocknungsmittels,
oder eine Kombination aus wenigstens zwei dieser Maßnahmen.

Geeignete Strip- oder Schleppgase sind z. B. Luft, Stickstoff, Kohlendioxid, Argon oder Mischungen davon. Geeignete Trocknungsmittel sind z. B. Molsiebe, Natriumsulfat, Magnesiumsulfat oder Silicagel.

Die destillative Entfernung des Wassers kann bei Normaldruck (ca. 1 atm = 1013,25 mbar) erfolgen. Sie kann auch bei Drücken über oder unter Normaldruck durchgeführt werden. Unter verringertem Druck (bezogen auf den Normaldruck) versteht man beispielsweise Druck im Bereich von 1 bis 1000 mbar. Bevorzugt werden Druckbereiche von 5 bis 500 mbar, insbesondere, 5 bis 200 mbar, insbesondere 5 bis 100 mbar, insbesondere 10 bis 100 mbar, insbesondere 10 bis 50 mbar, verwendet. Wesentlich ist, dass bei Drücken gearbeitet wird, die bei der jeweiligen Reaktionstemperatur kleiner sind als der Dampfdruck des Reaktionswassers.

Da die Stabilität von Enzymen nur in bestimmten Temperaturbereichen gewährleistet ist, muss die Reaktionstemperatur sorgfältig gewählt werden. Sie beträgt typischerweise 0 bis 100 °C, bevorzugt 20 bis 100 °C, besonders bevorzugt 20 bis 90 °C. Wie oben erwähnt, bedingt die gewählte Reaktionstemperatur das gegebenenfalls für den Austrag des Reaktionswassers anzulegende Vakuum.

Mit der Verwendung von Strip- oder Schleppgasen kann ebenso Austrag von Reaktionswasser erfolgen. Insbesondere in Kombination mit der destillativen Entfernung von Reaktionswasser lassen sich Ergebnisse erzielen, die sich insbesondere positiv auf die Ausbeute im Veresterungsprozess auswirken. Kritisch dabei sind die Stoffaustauschfläche und das Gasvolumen. Bevorzugt sind solche Gase, die weder mit den Reaktanden noch dem Katalysator, den Reaktormaterialien, geschweige denn dem Endprodukt reagieren, also inert sind. Der Fachmann ist mit dieser Verfahrensmaßnahme vertraut.

Ein spezielles geeignetes Verfahren zur enzymatischen Veresterung ist in der WO 2014/056756 beschrieben, worauf hier in vollem Umfang Bezug genommen wird.

Die nach dem erfindungsgemäßen Verfahren erhaltenen rohen Reaktionsgemische können wenigstens einem Aufarbeitungsschritt unterzogen werden. Dazu zählen z. B. Neutralisation, Aufreinigung und Trocknung. Eine Aufreinigung kann nach üblichen, dem Fachmann bekannten Methoden erfolgen, z. B. durch Extraktion und/oder Destillation.

Im Sinne der vorliegenden Erfindung werden bevorzugt Ester des THF-Glykols verwendet, die nach dem enzymatischen Verfahren hergestellt werden. Diese enthalten je nach eingesetzten Mengenverhältnissen Mono- und/oder Diester des THF-Glykols als Verbindungen der allgemeinen Formel (I).

Das Verhältnis von Mono- (R² steht für Wasserstoff) zu Diester des THF-Glykols (R2 steht für C(=O)R^{1'}) wurde nach der GC-Analyse bestimmt, die im Beispielsteil der vorliegenden Anmeldung offenbart wird.

Bevorzugt werden die Diester oder Mischungen von Monoester und Diester als Verbindungen der Formel (I) verwendet.

Sofern Mischungen von Mono:Diester verwendet werden, liegt das Verhältnis von Mischungen Monoester (R²= Wasserstoff) zu Diester (R²= C(=O)R^{1'}) in einem Verhältnis von 5:95 bis 35:65, vorzugsweise von 10:90 bis 30:70, wobei die Verhältnisse bestimmt wurden als Flächenverhältnis nach der schon erwähnten GC-Analyse im Beispielsteil.

Im Sinne der Erfindung werden vorzugsweise Verbindungen der Formel (I) verwendet, in der wenigstens einer der Reste R¹ und R^{1'} für einen linearen C13-C25-Alkylrest steht, vorzugsweise in der Rest R¹ bzw. die Reste R¹ und R^{1'} unabhängig voneinander für einen n-Tridecyl, n-Tetradecyl, n-Pentadecyl, n-Hexadecyl, n-Heptadecyl, n-Octadecyl, n-Nonadecyl, Arachinyl, Eicosyl, Heneicosyl, Isotridecyl, Isoheptadecyl- und/oder Hydroxyheptadecylrest, und deren Konstitutionsisomeren, stehen.

Die Alkylreste R¹ und R^{1'} stehen vorzugsweise für Alkylreste, die sich von großtechnisch verfügbaren Carbonsäuren oder Carbonsäuregemischen ableiten. In einer bevorzugten Ausführungsform sind R¹ und R^{1'} dann abgeleitete Alkylreste von natürlich vorkommenden Fettsäuren und Fettsäuregemischen, wie sie in der Natur aus pflanzlichen und/oder tierischen Ölen oder Fetten in Form von Triglyceriden zu gewinnen sind.

Geeignete Fettsäuren bzw. Fettsäuregemische sind Myristinsäure, Pentadecansäure, Palmitinsäure, Margarinsäure, Stearinsäure, Nonadecansäure, Tuberculostearinsäure, Arachinsäure, Behensäure und/oder Mischungen hiervon.

Typische Fette bzw. Triglyceride, die diese Fettsäuren bzw. Fettsäuregemische in hohen Mengen enthalten, sind Kokosfett und Palmöl und werden aus diesen großtechnisch gewonnen.

Ganz besonders bevorzugt werden im Sinne der Erfindung Verbindungen der Formel (I) verwendet, wobei wenigstens einer der Reste R¹ und R^{1'} für einen C15-C25 Alkylrest steht, der abgeleitet ist von einer C16-C24-Fettsäuremischung mit einem Gehalt an C18-C24 Fettsäure von 25 bis 100 Gew.-%, vorzugsweise 35 bis 99 Gew.-% und insbesondere 45 bis 99 Gew.-% - bezogen auf Fettsäuremischung. Insbesondere für die Verwendung geeignete Verbindungen sind solche der Formel (I), wobei beide Alkylreste R¹ und R^{1'} sich ableiten von einer C16-C24-Fettsäuremischung mit einem Gehalt an C18-C24 Fettsäure von 25 bis 100 Gew.-%, vorzugsweise 35 bis 99 Gew.-% und insbesondere 45 bis 99 Gew.-% - bezogen auf Fettsäuremischung.

Nach einer Ausführungsform der vorliegenden Erfindung werden Verbindungen der allgemeinen Formel (I) verwendet, in der R2 für C(=O)R^{1'} sowie R¹ und R^{1'} für einen Alkylrest mit 15 und 17 C-Atomen stehen (also ein Diester), vorzugsweise in einem Gewichtsverhältnis C15:C17 von 50:50 steht, d.h. THF-Glykoldipalmitatstearat.

Einer besonders bevorzugten Ausführungsform der vorliegenden Erfindung entsprechend werden Verbindungen verwendet, in der R2 für C(=O)R^{1'} steht und R¹ sowie R^{1'} für einen C17-C25 Alkylrest stehen, der sich von einer C16-C24-Fettsäuremischung ableitet, die 45 bis 99 Gew.-% C18-C24-Fettsäure enthält, d.h. praktisch THF-glykoldistearat und THF-glykoldibehenat.

### Erfindungsgemäße Perlglanzkonzentrate

Die erfindungsgemäß verwendeten Verbindungen der allgemeinen Formel (I) können in die wässrigen, tensidischen Zubereitungen direkt als Perlglanzwachs zugegeben werden.

Vorzugsweise werden sie aber als Perlglanzkonzentrate formuliert, die dann den wässrigen, tensidischen Zubereitungen zugegeben werden können.

Ein weiterer Gegenstand der vorliegenden Erfindung sind demgemäß Perlglanzkonzentrate für wässrige, tensidische Zubereitungen enthaltend
(a) 7,5 bis 35 Gew.-% Ester des 2,5-Di(hydroxymethyl)tetrahydrofurans der allgemeinen Formel (I)
(b) 0,1 bis 60 Gew-% anionische, nichtionische, kationische, ampholytische oder zwitterionische Tenside oder deren Kombinationen
(c) 0 - 40 Gew.-% Polyole
(d) ad 100 Gew.-% Wasser.

Typische Beispiele für anionische Tenside sind Seifen, Alkylsulfonate, Alkylbenzolsulfonate, Olefinsulfonate, Alkylethersulfonate, Glycerinethersulfonate, Methylestersulfonate, Sulfofettsäuren, Alkylsulfate, Ethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acylglutamate und Acylaspartate, sowie Acyllactylate, Acyltartrate, Alkyloligoglucosidsulfate, Alkylglucosecarboxylate, Proteinfettsäurekondensate und Alkyl(ether)phosphate.

Geeignete Seifen sind z. B. Alkali-, Erdalkali- und Ammoniumsalze von Fettsäuren, wie Kaliumstearat.

Bevorzugte Alkylsulfate sind Sulfate von Fettalkoholen der allgemeinen Formel R³-O-SO₃Y¹, worin R³ für einen linearen oder verzweigten, gesättigten oder ungesättigten Kohlenwasserstoffrest mit 6 bis 22 Kohlenstoffatomen und Y¹ für ein Alkalimetall, das einwertige Ladungsäquivalent eines Erdalkalimetalls, Ammonium-, Mono-, Di-, Tri- oder Tetraalkylammonium, Alkanolammonium oder Glucammonium steht. Geeignete Fettalkoholsulfate werden vorzugsweise durch Sulfatierung von nativen Fettalkoholen oder synthetischen Oxoalkoholen und nachfolgender Neutralisation erhalten werden. Typische Beispiele für Fettalkoholsulfate sind die Sulfatierungsprodukte des Capronalkohols, Caprylalkohols, 2-Ethylhexylalkohols, Caprinalkohols, Laurylalkohols, Isotridecylalkohols, Myristylalkohols, Cetylalkohols, Palmoleylalkohols, Stearylalkohols, Isostearylalkohols, Oleylalkohols, Elaidylalkohols, Petroselinylalkohols, Linolylalkohols, Linolenylalkohols, Behenylalkohols und Elaeostearylalkohols sowie die Salze und Mischungen davon. Bevorzugte Salze der Fettalkoholsulfate sind die Natrium- und Kaliumsalze, insbesondere die Natriumsalze. Bevorzugte Mischungen der Fettalkoholsulfate basieren auf technischen Alkoholmischungen, die z. B. bei der Hochdruckhydrierung von technischen Methylestern auf Basis von Fetten und Ölen oder bei der Hydrierung von Aldehyden aus der Oxosynthese oder bei der Dimerisierung von ungesättigten Fettalkoholen anfallen. Vorzugsweise werden zur Herstellung von Alkylsulfaten Fettalkohole und Fettalkoholgemische mit 12 bis 18 Kohlenstoffatomen und insbesondere 16 bis 18 Kohlenstoffatomen eingesetzt. Typische Beispiele hierfür sind technische Alkoholsulfate auf Basis von pflanzlichen Rohstoffen.

Geeignete Olefinsulfonate werden z. B. durch Anlagerung von SO₃ an Olefine der Formel R⁴-CH=CH-R⁵ und nachfolgende Hydrolyse und Neutralisation erhalten, wobei R⁴ und R⁵ unabhängig voneinander für H oder Alkylreste mit 1 bis 20 Kohlenstoffatomen stehen, mit der Maßgabe, dass R⁴ und R⁵ zusammen mindestens 6 und vorzugsweise 8 bis 20, speziell 10 bis 16, Kohlenstoffatome aufweisen. Die Olefinsulfonate können als Alkali-, Erdalkali-, Ammonium-, Alkylammonium-, Alkanolammonium- oder Glucammoniumsalze vorliegen. Vorzugsweise liegen die Olefinsulfonate als Natriumsalze vor. Das hydrolysierte alpha-Olefinsulfonierungsprodukt, d.h. die alpha-Olefinsulfonate setzen sich aus ca. 60 Gew.-% Alkansulfonaten und ca. 40 Gew.-% Hydroxyalkansulfonaten zusammen; hiervon sind etwa 80 bis 85 Gew.-% Mono- und 15 bis 20 Gew.-% Disulfonate.

Bevorzugte Methylestersulfonate (MES) werden durch Sulfonierung der Fettsäuremethylester von pflanzlichen oder tierischen Fetten oder Ölen gewonnen. Bevorzugt sind Methylestersulfonate aus pflanzlichen Fetten und Ölen, z. B. aus Rapsöl, Sonnenblumenöl, Sojaöl, Palmöl, Kokosfett, etc.

Eine bevorzugte Klasse anionischer Tenside sind die Ethersulfate. Ethersulfate (Alkylethersulfate) stellen bekannte anionische Tenside dar, die großtechnisch durch SO₃- oder Chlorsulfonsäure (CSA)-Sulfatierung von Fettalkohol- oder Oxoalkoholpolyglycolethern und nachfolgende Neutralisation hergestellt werden können.

Bevorzugt sind insbesondere Fettalkoholethersulfate der allgemeinen Formel R⁶O-(CH₂CH₂O)ₘSO₃Y²,
in der R⁶ für einen linearen oder verzweigten Alkyl- und/oder Alkenylrest mit 6 bis 22 Kohlenstoffatomen, m für Zahlen von 1 bis 10 und Y² für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Iso-tridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C_{12/14}- bzw. C_{12/18}- Kokosfettalkoholfraktionen in Form ihrer Natrium- und/oder Magnesiumsalze.

Bevorzugte Sarkosinate sind Natriumlauroylsarkosinat oder Natriumstearoylsarkosinat.

Bevorzugte Proteinfettsäurekondensate sind pflanzliche Produkte auf Weizenbasis.

Bevorzugte Alkylphosphate sind Mono- und Diphosphorsäurealkylester.

Geeignete Acylglutamate sind Verbindungen der Formel (II), 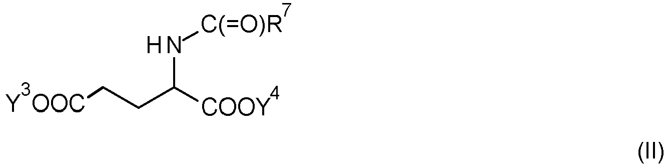 worin COR⁷ für einen linearen oder verzweigten Acylrest mit 6 bis 22 Kohlenstoffatomen und 0, 1, 2 oder 3 Doppelbindungen und Y³ und Y⁴ unabhängig voneinander für Wasserstoff, ein Alkalimetall, das einwertige Ladungsäquivalent eines Erdalkalimetalls, Ammonium, Alkylammonium, Alkanolammonium oder Glucammonium steht. Die Herstellung von Acylglutamaten erfolgt beispielsweise durch Schotten-Baumann-Acylierung von Glutaminsäure mit Fettsäuren, Fettsäureestern oder Fettsäurehalogeniden. Acylglutamate sind kommerziell beispielsweise von der BASF SE, Clariant AG, Frankfurt/DE, oder der Ajinomoto Co. Inc., Tokio/JP, erhältlich. Eine Übersicht zu Herstellung und Eigenschaften der Acylglutamate findet sich von M. Takehara et al. in J. Am. Oil Chem. Soc. 49 (1972) 143**.** Typische als Komponente b) geeignete Acylglutamate leiten sich bevorzugt von Fettsäuren mit 6 bis 22 und besonders bevorzugt 12 bis 18 Kohlenstoffatomen ab. Es werden insbesondere die Mono- oder Dialkalimetallsalze des Acylglutamats eingesetzt. Dazu zählen z. B. (Handelsnamen der Fa. Ajinomoto, USA in Klammern): Natriumcocoylglutamat (Amisoft® CS-11), Dinatriumcocoylglutamat (Amisoft® ECS-22SB), Triethanolammoniumcocoylglutamat (Amisoft® CT-12), Triethanolammoniumlauroylglutamat (Amisoft® LT-12), Natriummyristoylglutamat (Amisoft MS-11), Natriumstearoylglutamat (Ami-soft® HS-11 P) und Mischungen davon.

Zu den nichtionischen Tensiden gehören beispielsweise:
- Fettalkoholpolyoxyalkylenester, beispielsweise Laurylalkoholpolyoxyethylenacetat,
- Alkylpolyoxyalkylenether, die sich von niedermolekularen C₁-C₆-Alkoholen oder von C₇-C₃₀-Fettalkoholen ableiten. Dabei kann die Etherkomponente aus Ethylenoxideinheiten, Propylenoxideinheiten, 1,2-Butylenoxideinheiten, 1,4-Butylenoxideinheiten und statistischen Copolymeren und Blockcopolymeren davon abgeleitet sein. Dazu zählen speziell Fettalkoholalkoxylate und Oxoalkoholalkoxylate, insbesondere vom Typ
   RO-(R⁸O)ᵣ(R⁹O)ₛR¹⁰ mit R⁸ und R⁹ unabhängig voneinander = C₂H₄, C₃H₆, C₄H₈ und R¹⁰ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie iso-Tridecylalkohol- und Oleylalkoholpolyoxyethylenether,
- Alkylarylalkohol-polyoxyethylenether, z. B. Octylphenol-polyoxyethylenether,
- Zuckertenside, bevorzugt Alkylpolyglycoside, Sorbitester, wie beispielsweise Sorbitanfettsäureester (Sorbitanmonooleat, Sorbitantristearat), Polyoxyethylensorbitanfettsäureester, N-Alkylgluconamide,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Alkylmethylsulfoxide,
- Alkyldimethylphosphinoxide, wie beispielsweise Tetradecyldimethylphosphinoxid.

Eine bevorzugte Klasse nichtionischer Tenside sind die Zuckertenside, speziell Alkyl(poly)glycoside. Im Sinne der Erfindung wird der Begriff Alkyl(poly)glycoside synonym zu Alkyl(oligo)glycoside verwendet und auch mit der Abkürzung "APG" bezeichnet. Alkylglycoside und/oder Alkylpolyglycoside umfassen sowohl Alkyl- als auch Alkenyl(poly)glycoside und haben bevorzugt die Formel R¹¹O-[G]ₚ, in der R¹¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p gibt den Polymerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylpolyglykosid eine analytisch ermittelte rechnerische Größe, die meistens eine gebrochene Zahl darstellt. Vorzugsweise werden Alkyl- und/oder Alkenylpolyglykoside mit einem mittleren Polymerisierungsgrad p von 1,1 bis 3,0 eingesetzt. Aus anwendungstechnischer Sicht sind solche Alkyl- und/oder Alkenylpolyglykoside bevorzugt, deren Polymerisierungsgrad kleiner als 1,7 ist und insbesondere zwischen 1,2 und 1,4 liegt.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate. Beispielsweise können Cocodimethylsulfopropylbetain, Laurylbetain, Cocamidopropylbetain, Natriumcocamphopropionat oder Tetradecyldimethylaminoxid eingesetzt werden.

Zu den geeigneten kationischen Tensiden gehören quaternierte Ammoniumverbindungen, insbesondere Alkyltrimethylammonium- und Dialkyldimethylammoniumhalogenide und -alkylsulfate sowie Pyridin- und Imidazolin-Derivate, insbesondere Alkylpyridinium-Halogenide. Beispielsweise können Behenyl oder Cetyltrimethylammoniumchlorid eingesetzt werden. Geeignet sind weiterhin sogenannte Esterquats, die auf quartären Triethanol-Methyl-Ammonium- oder quartären Diethanol-Dimethyl-Ammonium-Verbindungen mit langen Kohlenwasserstoffketten in Form von Fettsäureestern basieren. Dazu zählt beispielsweise Bis(acyloxyethyl)hydroxyethylammonium Methosulfat. Geeignet ist weiterhin Dehyquart L 80 (INCI: Dicocoylethyl Hydroxyethylmonium Methosulfate (and) Propylene Glycol).

Einer Ausführungsform entsprechend enthalten die erfindungsgemäßen Perlglanzkonzentrate anionische oder nichtionische Tenside vorzugsweise anionische Tenside ausgewählt aus der von Fettalkoholethersulfaten gebildeten Gruppe oder nichtionische Tenside, ausgewählt aus der von Alkyl(poly)glycosiden gebildeten Gruppe.

Einer weiteren Ausführungsform entsprechend enthalten die erfindungsgemäßen Perlglanzkonzentrate eine Kombination von amphoteren bzw. zwitterionischen Tensiden und nichtionischen Tensiden, vorzugsweise eine Kombination von Betainen und Alkylpolyoxyalkylenether, insbesondere eine Kombination von Alkylamidopropylbetainen und Fettalkoholalkoxylaten.

Insbesondere enthalten die erfindungsgemäßen Perlglanzkonzentrate die Ester der Formel (I) in Mengen von 15 bis 30 Gew.-%, insbesondere von 20 bis 30 Gew.-%.

Die bevorzugten Perlglanzkonzentrate enthalten als b) anionische Tenside oder nichtionische Tenside oder Kombinationen von zwitterionischen und nichtionischen Tensiden, vorteilhafterweise in Mengen von 10 bis 30 Gew.-%.

Optional können Polyole in den erfindungsgemäßen Konzentraten enthalten sein, vorzugsweise in Mengen von 0,1 bis 20, insbesondere 0,2 bis 15 Gew.-% -bezogen auf Perlglanzkonzentrat. Polyole, die hier in Betracht kommen, besitzen vorzugsweise 2 bis 15 Kohlenstoffatome und mindestens zwei Hydroxylgruppen. Insbesondere geeignete Polyole sind Glycerin und/oder Sorbitol.

Des Weiteren ist noch Wasser ad 100 Gew.-% enthalten.

Die Perlglanzkonzentrate können erfindungsgemäß hergestellt werden, indem man
- die Ester des 2,5-Di(hydroxymethyl)tetrahydrofurans der allgemeinen Formel (I) sowie die Tenside (b) sowie ggf. die Polyole (c) und ad 100 Gew.-% Wasser unter Rühren auf Temperaturen erwärmt, die 5 bis 20 °C über dem Schmelzpunkt des Esters (a) liegen,
- die Mischung bei diesen Temperaturen rührt und
- anschließend die Mischung unter stetigem Rühren auf etwa Raumtemperatur (20 bis 23 °C) abkühlt.

Es ist auch möglich die Perlglanzkonzentrate herzustellen, in dem man
- zu einer wässrigen Paste der Tenside (b) die Ester des 2,5-Di(hydroxymethyl)tetrahydrofurans der allgemeinen Formel (I) einrührt und auf Temperaturen, die 5 bis 20 °C über dem Schmelzpunkt des Esters liegen, erwärmt,
- die Mischung bei diesen Temperaturen rührt und
- anschließend mit weiterem Wasser und ggf. Polyolen die gewünschte Konzentration einstellt.

In der Regel empfiehlt sich eine Rührzeit der Mischung von etwa 15 bis 60 Minuten, bevor vorzugsweise die Mischung mit einer Kühlrate von etwa 10 bis 30 °C, vorzugsweise 15 bis 25 °C pro Stunde abgekühlt wird.

Die erfindungsgemäßen Perlglanzkonzentrate sind fließfähige Mischungen, die leicht in wässrige, tensidische Zubereitungen eingerührt werden können und dort einen brillanten Glanz mit hohem Weißgrad erzeugen.

Demgemäß ist ein weiterer Gegenstand der vorliegenden Erfindung die Verwendung der beschriebenen Perlglanzkonzentrate zur Herstellung von wässrigen, tensidischen Zubereitungen mit Perlglanzeffekt.
Vorzugsweise werden die Perlglanzkonzentrate in Mengen von 0,2 bis 10 Gew.-% - bezogen auf Zubereitung - verwendet.

### Wässrige, tensidische Zubereitungen

Weitere Gegenstände der Erfindung beziehen sich auf die wässrigen, tensidischen Zubereitungen mit Perlglanzeffekt, die 0,2 bis 10 Gew.-% des oben beschriebenen Perlglanzkonzentrats enthalten bzw. 0,015 bis 3,5 Gew.-% Ester des 2,5-Di(hydroxymethyl)tetrahydrofurans der allgemeinen Formel (I) - bezogen auf Zubereitung.

Einer Ausführungsform entsprechend handelt es sich bei den erfindungsgemäßen wässrigen, tensidischen Zubereitungen um Reinigungsmittel für Geschirr, insbesondere Handgeschirrspülmittel.

In einer speziellen Ausführungsform handelt es sich bei den erfindungsgemäßen Zubereitungen um eine wässrige, tensidische kosmetische Zubereitung, vorzugsweise für die Haut und/oder die Haare.

Aufgrund ihrer Eigenschaft als Perlglanzwachs eignen sich die zuvor beschriebenen Verbindungen der Formel (I) bzw. deren Konzentrate vorzugsweise in Form eines Wasch-, Dusch- oder Badepräparats von flüssiger bis gelförmiger Konsistenz für Haut und/oder Haar, insbesondere in Haarshampoos mit oder ohne konditionierende Wirkung.

Dementsprechend enthält die wässrige tensidische, kosmetische Zubereitung neben der Verbindung der allgemeinen Formel (I), wie zuvor definiert, bzw. des Perlglanzkonzentrats wie zuvor definiert, wenigstens einen davon verschiedenen kosmetischen oder pharmazeutischen Wirkstoff und/oder Hilfsstoff.

Je nach Applikationszweck können enthalten sein: oberflächenaktive Substanzen (Tenside, Emulgatoren), Ölkörper, weitere Perlglanzwachse, Konsistenzgeber, Verdickungsmittel, Überfettungsmittel, Stabilisatoren, Polymere, Siliconverbindungen , Fette, Wachse, Lecithine, Phospholipide, biogene Wirkstoffe, UV-Licht-schutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Die Mengen der jeweiligen Zusätze richten sich nach der beabsichtigten Verwendung.

Als oberflächenaktive, tensidische Substanz können anionische, nichtionische, kationische und/oder amphotere bzw. zwitterionische Tenside bzw. Emulgatoren oder ein beliebiges Gemisch dieser Tenside/Emulgatoren enthalten sein, beispielsweise wie bereits die im Zusammenhang mit den Perlglanzkonzentraten beschriebenen Tenside. Der Gehalt hängt von der Art der Formulierung ab und liegt üblicherweise zwischen 3 - 20 Gew.-%. In tensidhaltigen kosmetischen Zubereitungen, wie beispielsweise Duschgelen, Schaumbädern, Shampoos etc. ist vorzugsweise wenigstens ein anionisches Tensid, insbesondere ein Fettalkoholethersulfat der beschriebenen Art, enthalten.

### Ölkörper

Die neueren Duschgele mit Körperpflegemittel, wie Cremes, Lotionen und Milchen, enthalten üblicherweise Ölkörper und Emollients, die dazu beitragen, die sensorischen Eigenschaften weiter zu optimieren. Die Ölkörper sind üblicherweise in einer Gesamtmenge von 1 - 50 Gew.-%, vorzugsweise 5 - 25 Gew.-% und inbesondere 5 - 15 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, Guerbetcarbonate auf Basis von Fettalkoholen mit 6 bis 18, vorzugsweise 8 bis 10 C-Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE) oder Ringöffnungsprodukte von epoxidierten Fettsäureestern mit Polyolen.

### Fette und Wachse

Auch Fette und Wachse können als Pflegestoffe enthalten sein und auch, um die Konsistenz der Kosmetika zu erhöhen. Typische Beispiele für Fette sind Glyceride, d. h. feste oder flüssige pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage.

Des weiteren können Verdickungsmittel enthalten sein, beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyacrylate, (z. B. Carbopole® und Pemulen-Typen von Goodrich; Synthalene® von Sigma; Keltrol-Typen von Kelco; Sepigel-Typen von Seppic; Salcare-Typen von Allied Colloids und Cosmedia® SP und SPL von Cognis), Polyacrylamide, Polymere, Polyvinylalkohol, Polyvinylpyrrolidon und Bentonite wie z. B. Bentone® Gel VS-5PC (Rheox). Weiter in Frage kommen Elektrolyte wie Kochsalz und Ammoniumchlorid.

Als Stabilisatoren können Metallsalze von Fettsäuren, wie z. B. Magnesium-, Aluminium- und/oder Zinkstearat bzw. -ricinoleat eingesetzt werden.
Weitere mögliche Inhaltsstoffe sind UV-Lichtschutzfilter und Antioxidantien. Unter UV-Licht-schutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol® 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide bzw. Salze in Frage. Beispiele für geeignete Metalloxide sind insbesondere Zinkoxid und Titandioxid. Als Salze können Silicate (Talk), Bariumsulfat oder Zinkstearat eingesetzt werden. Die Oxide und Salze werden in Form der Pigmente für hautpflegende und hautschützende Emulsionen verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Als biogene Wirkstoffe können beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe enthalten sein

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

Geeignete hyperemisierend wirkende Stoffe, welche die Durchblutung der Haut anregen, sind z. B. ätherische Öle, wie Latschenkiefer, Lavendel, Rosmarin, Wacholderbeere, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Zusammensetzungen können als kosmetischen Wirkstoff (wie auch gegebenenfalls als Hilfsstoff) wenigstens ein kosmetisch akzeptables Polymer enthalten. Dazu zählen ganz allgemein anionische, kationische, amphotere und neutrale Polymere.

Zur Einstellung bestimmter Eigenschaften, wie z. B. Verbesserung des Anfassgefühls bzw. der konditionierenden Eigenschaften können die Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die wässrigen tensidischen, kosmetischen Zubereitungen enthalten auch Wasser, vorzugsweise in Mengen über 5 Gew.-%, vorzugsweise von 20 bis 90 Gew.-% - bezogen auf Zubereitung.

### Beispiele

### GC-Analyse zur Bestimmung des Verhältnisses von Mono- zu Diester der THF-Glycolester

In Vorbereitung der GC-Analyse wurden die Proben mit N-Methyl-N-(trimethylsilyl)trifluoracetamid (MSTFA) silyliert. Dafür wurden 10 µL Probe mit 500 µL MSTFA und 500 µL Isooktan gemischt und die Derivatisierung für 2 Stunden bei Raumtemperatur durchgeführt.
Die gaschromatographische Auftrennung erfolgte auf einem HP 6890 Gaschromatographen: Detektor: FID, 410°C; Säule: Zebron ZB-5HT (Phenomenex), 15 m x 0,25 mm x 0,10 µm; Trägergas: Wasserstoff, 4.0 mL/min; Temperaturprogramm: 1 min bei 50°C, 35°C/min auf 100°C, 4 min bei 100°C, 12°C/min auf 250°C, 20 min 250°C, 50°C/min auf 380°C, 8.4 min bei 380°C.

Typische Retentionszeiten der THF-Glycol Ester:

| | |
|---|---|
| THF-Glycol Palmitat | 17.4 min |
| THF-Glycol Stearat | 18.8 min |
| THF-Glycol Behenat | 21.8 min |
| THF-Glycol Dipalmitat | 40.4 min |
| THF-Glycol Palmitat/Stearat | 40.9 min |
| THF-Glycol Distearat | 41.2 min |
| THF-Glycol Stearat/Behenat | 41.6 min |
| THF-Glycol Dibehenat | 42.0 min |

### A) Herstellung der 2,5-Di(hydroxymethyl)tetrahydrofurandiester (verkürzt THF-Glykol-Diester)

### Beispiel 1: Enzymatische Synthese von 2,5-Di(hydroxymethyl)tetrahydrofuranstearat (=THF-Glykoldistearat) mit Monoester : Diester = 2 : 98 % ; Fettsäure > 98 Gew.-% C18

568 g Stearinsäure (> 98 Gew.-% C18; Merck) wurde mit 134 g 2,5-Di(hydroxymethyl)tetra-hydrofuran in einem 1 l Rührreaktor auf 75 °C temperiert und vermischt. Die Veresterungsreaktion wurde durch Zugabe von 1 Gew.-% kommerziell erhältlichem Novozym® 435 (Lipase B aus *Candida antarctica* immobilisiert auf einem Polymethacrylatträger) gestartet. Die Entfernung des entstehenden Reaktionswassers erfolgte durch eine Kombination aus verringertem Druck (50 bis 10 mbar) und Begasung mit Stickstoff als Stripgas (0 bis 11 NL/h*kg). Der Verlauf der Reaktion wurde mittels Säurezahl (ISO 660) verfolgt bis eine Säurezahl von ≤ 1 erreicht wurde. Am Ende der Reaktionen wurde der Katalysator mittels Filtration vom Produkt abgetrennt.
Das Produkt hatte einen Schmelzpunkt von: 55°C (ISO 6321). Das Mono- zu Diesterverhältnis wurde mittels der Flächenverhältnisse in der GC-Analyse bestimmt (siehe oben).

### Beispiel 2 : Enzymatische Synthese von 2,5-Di(hydroxymethyl)tetrahydrofuranstearat (=THF-Glykoldistearat) mit Monoester : Diester = 30% : 70% ; Fettsäure > 98 Gew.-%C18

Analog Beispiel 1 wurden 300 g Stearinsäure (ca. 98,5 Gew.-% C18; Merck), mit 100 g 2,5-Di(hydroxymethyl)tetrahydrofuran umgesetzt. Der Verlauf der Reaktion wurde mittels Säurezahl (ISO 660) verfolgt bis eine Säurezahl von ≤ 1 erreicht wurde. Das Produkt hatte einen Schmelzpunkt von 52 °C (ISO 6321).
Das Mono- zu Diesterverhältnis wurde mittels der Flächenverhältnisse in der GC-Analyse bestimmt (siehe oben).

### Beispiel 3: Enzymatische Synthese eines 2,5-Di(hydroxymethyl)tetrahydrofuran-diesters einer Fettsäuremischung mit Behensäure mit Monoester : Diester = 8% : 92% ; Fettsäure ca. 60 Gew.-% C22; ca. 30 Gew.-% C18

330 g einer technischen Fettsäuremischung (ca. 60% Behensäure ca. 30% Stearinsäure, ca. 5% Arachinsäure Palmitinsäure, Lignocerinsäure jeweils < 2 Gew. %) wurden mit 70 g 2,5-Di(hydroxymethyl)tetrahydrofuran in einem 1 l Rührreaktor auf 85 °C temperiert und vermischt. Die Veresterungsreaktion wurde durch Zugabe von 1 Gew.-% kommerziell erhältlichem Novozym® 435 gestartet. Die Entfernung des entstehenden Reaktionswassers erfolgte durch die Kombination aus verringertem Druck (50 bis 10 mbar) und Begasung mit Stickstoff als Stripgas (0 bis 11 NL/h*kg). Der Verlauf der Reaktion wurde mittels Säurezahl (ISO 660) verfolgt bis eine Säurezahl von ≤ 1 erreicht wurde. Am Ende der Reaktionen wurde der Katalysator mittels Filtration vom Produkt abgetrennt.
Das Produkt hatte einen Schmelzpunkt von: 52°C (ISO 6321), das Mono- zu Diesterverhältnis wurde mittels der Flächenverhältnisse in der GC-Analyse bestimmt (siehe oben).

### Beispiel 4: Enzymatische Synthese von 2,5-Di(hydroxymethyl)tetrahydrofuranpalmitat/stearat (=THF-Glykol-dipalmitat/stearat) mit Monoester : Diester = 9% : 91% ; Fettsäure ca. 50 Gew.-% C18 und 50 Gew.-% C16

2760 g Edenor HPA® (50% Stearinsäure, 50% Palmitinsäure) wurden mit 730 g 2,5-Di(hydroxymethyl)tetrahydrofuran in einem 5 l Rührreaktor auf 75 °C temperiert und vermischt. Die Veresterungsreaktion wurde durch Zugabe von 1 Gew.-% kommerziell erhältlichem Novozym® 435 gestartet. Die Entfernung des entstehenden Reaktionswassers erfolgte durch die Kombination aus verringertem Druck (50 bis 10 mbar) und Begasung mit Stickstoff als Stripgas (0 bis 11 NL/h*kg). Der Verlauf der Reaktion wurde mittels Säurezahl (ISO 660) verfolgt bis eine Säurezahl von ≤ 1 erreicht wurde. Am Ende der Reaktionen wurde der Katalysator mittels Filtration vom Produkt abgetrennt.
Das Produkt hatte einen Schmelzpunkt von: 45°C (ISO 6321), das Mono- zu Diesterverhältnis wurde mittels der Flächenverhältnisse in der GC-Analyse bestimmt (siehe oben).

### B) Tensidische Basis-Formulierung zur Bestimmung des Perlglanzes

Es wurde folgende Basis-Formulierung (Tabelle 1a) zur Bestimmung des Perlglanzes hergestellt (* = Handelsprodukt der BASF Personal Care & Nutrition GmbH sowie INCI-Bezeichnung; % Angaben sind Gew.-% an Aktivsubstanz

| | |
|---|---|
| Deion. Wasser | 84.7 % |
| Sodium Laureth Sulfate; Texapon N 70 ® * | 8.6 % |
| Coco-Glucoside Plantacare 8/18®* | 1.6 % |
| Cocoamidopropyl Betaine Dehyton PK45®* | 1.4 % |
| Sicomet amaranth (1 Gew.-%ig; in Wasser) Colour Index 16185 | 0.1 % |
| 1,2 Dibromo-2,4dicyanobutan and 2-phenoxyethanol; | 0.1 % |
| Euxyl K 400®; Fa. Schülke Konservierungsmittel | |
| NaCl | 2.5 % |
| Erfindungsgemäßer THF-Glykoldiester (=Beispiel 1 bis 4)) | 1 % |

Zur Herstellung der Basis-Formulierung wurden alle Bestandteile außer dem erfindungsgemäß verwendeten THF-Glykoldiester vorgelegt und homogen miteinander verrührt. Unter Rühren bei 80 UpM wurde die Mischung auf 80 °C erhitzt. Nachdem 80 °C erreicht wurde, wurden die aufgeschmolzenen erfindungsgemäß verwendeten THF-Glykoldiester zugeben und etwa 30 Minuten bei 80 °C nachgerührt.

Anschließend wurde die Formulierung bei einer Rührgeschwindigkeit von 80rpm und mit einer Kühlrate von ca. 20 °C /Stunde auf Raumtemperatur (20-23 °C) abgekühlt.

Die Beurteilung des Perlglanzeffektes wurde nach 24 Stunden vorgenommen.
Als Benchmark (=Vergleich) diente das auf dem Markt als Perlglanzmittel Standard mäßig verwendete Ethylenglykoldistearat (C16/C18=50%/50%).

Ergebnisse Perlglanzmessung (Optische Bestimmung des Perlglanzes und Weißgrades in Schulnoten 1-6 (1 ist die beste Note) in Tabelle 1b):

**Tabelle 1b) Perlglanz der THF-Glykolester in der Tensidischen Formulierung nach Tabelle 1a)**

| **Ester für den Perlglanz** | **Monoester:Diester in % nach obiger GC-methode** | **Wei ss grad** | **Perlglanz** |
|---|---|---|---|
| Benchmark = Ethylenglycoldistearate (C16/C18=50%/50%) | 10 / 90 | 1 | 1 |
| THF-Glykol Distearat nach Beispiel 1 | 2 / 98 | 1 | 1 |
| THF-Glykol Distearat nach Beispiel 2 | 31 / 69 | 2 | 2 |
| THF-Glykol Dipalmitatstearat (C16/C18=50%/50%) nach Beispiel 4 | 9 / 91 | 2 | 2 |
| THF-Glykol Dibehenat nach Beispiel 3 | 22 /78 | 1 | 1 |

Perlglanzkategorie: 1 = am besten; optische Beurteilung durch Vergleich mit dem Benchmark als Standard-Perlglanzmittel.

### C) Tensidische Perlglanzkonzentrate mit THF-Glykol-ester + Haarshampoos mit Perlglanzkonzentrat, Mengenangaben sind Gew.-%;

Es wurden Perlglanzkonzentrate gemäß Tabelle 2a hergestellt durch Vermischen aller Bestandteile gemäß Basis-Formulierung B. Dazu wurde die Mischung 30 Minuten unter Rühren auf 80°C erwärmt und anschließend mit einer Kühlrate von 15 °C pro Stunde abgekühlt.

**Tabelle 2a) Perlglanzkonzentrat**

| Zusammensetzung | Benchmark | R2 | R3 | R4 | Benchmark | R6 | R7 |
|---|---|---|---|---|---|---|---|
| Ethylenglyokldistearate (C16/C18=50%/50%); Cutina AGS®* | 25 | - | - | - | 25 | - | - |
| THF-Glykol Distearat nach Beispiel 1 | - | 25 | - | - | - | 25 | - |
| THF-Glykol Dibehenat nach Beispiel 3 | - | - | 25 | - | - | - | - |
| THF-Glvkoldipalmitatstearat nach Beispiel 4 | - | - | - | 25 | - | - | 25 |
| Ameisensäure 60% ig | 0.6 | 0.6 | 0.6 | 0.6 | - | - | - |
| Kokosalkohol+4EO Dehydol LS4 (=nichtionisches Tensid) | 11 | 11 | 11 | 11 | - | - | - |
| Kokosalkylglucosid Plantacare 8/18®*(=nichtionisches Tensid) | - | - | - | - | 25 | 25 | 25 |
| Kokosfettsäurebetain Dehyton PK45®* (= zwitterionisches Tensid) | 8 | 8 | 8 | 8 | - | - | - |
| Glycerin(=Polyol) | 0.5 | 0.5 | 0.5 | 0.5 | 10 | 10 | 10 |
| Wasser | ad 100 | | | | | | |
| Viskosität der Konzentrate nach Brookfield Spindel 5, 10 rpm [mPas] | | | | | | | |
| - nach Herstellung, 23°C | 5.000 | 8.000 | 7.500 | 9.000 | 4.000 | 6.000 | 7.000 |

Die Perlglanzkonzentrate wurden dann in wässrige Haarshampooformulierungen eingebracht (siehe Tabelle 2b) durch Vermischen der folgenden Bestandteile bei 25 °C:

| | |
|---|---|
| 4 | Gew.-% der Perlglanzkonzentrate, |
| 15 | Gew.-% Texapon N 70 ®* (Kokosfettalkohol+2EO-sulfat-Natriumsalz) |
| 3 | Gew.-% Dehyton PK45 ®* (Kokosfettsäurebetain, zwitterionisches Tensid) |
| 1,5 | Gew.-% Natriumchlorid und |
| 1,5 | Gew.-% Plantacare 8/18® (=Kokosalkylglucosid; nichtionisches Tensid) |

sowie ad 100 Gew.-% Wasser und Konservierungsmittel.

Die Beurteilung des Perlglanzes erfolgte optisch auf einer Skala von 1 = brillant bis 5 = stumpf; der Weißgrad wurde ebenfalls optisch auf einer Skala 1-5 visuell bestimmt und mit 1 = sehr weiß oder 5 = wenig weiß beurteilt, wobei 1 die beste Note ist.

**Tabelle 2b) Haarshampoo mit Perlglanzkonzentrat**

| Haarshampoo mit Perlglanzkonzentrat | Benchmark | R2 | R3 | R4 | Benchmark | R6 | R7 |
|---|---|---|---|---|---|---|---|
| - Brillanz | 1 | 1 | 1 | 2 | 1.5 | 1.5 | 2.0 |
| - Weißgrad | 2 | 2 | 1.5 | 2.5 | 2.5 | 2.5 | 2.5 |

## Patentansprüche

1. Verwendung von Estern des 2,5-Di(hydroxymethyl)tetrahydrofurans der allgemeinen Formel (I), 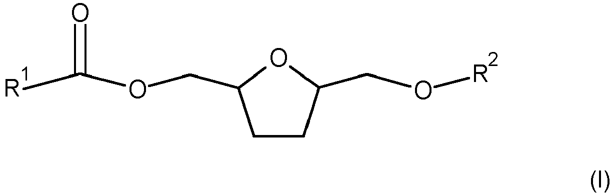 worin
R² für C(=O)R^{1'} steht und wobei R¹ und R^{1'} unabhängig voneinander für einen C13-C25-Alkylrest steht, der ggf. mit einer Hydroxylgruppe substituiert ist
oder
R² für Wasserstoff steht und R¹ für einen C13-C25-Alkylrest, der ggf. mit einer Hydroxylgruppe substituiert ist, oder Mischungen derartiger Ester
als Perlglanzwachs in wässrigen, tensidischen Zubereitungen.

2. Verwendung von Estern der allgemeinen Formel (I) nach Anspruch 1, wobei wenigstens einer der Reste R¹ und R^{1'} für einen linearen C13-C25-Alkylrest steht.

3. Verwendung von Estern der allgemeinen Formel (I) nach Anspruch 1 oder 2, wobei wenigstens einer der Reste R¹ und R^{1'} für einen C15-C25-Alkylrest steht, der abgeleitet ist von einer C16-C24-Fettsäuremischung mit einem Gehalt an C18-C24 Fettsäure von 25 bis 100 Gew.-%, vorzugsweise 35 bis 99 Gew.-% und insbesondere 45 bis 99 Gew.-% - bezogen auf Fettsäuremischung.

4. Verwendung von Estern der allgemeinen Formel (I) nach einem der Ansprüche 1 bis 3, wobei R² für C(=O)R^{1'} steht und die Reste R¹ und R^{1'} für einen C17-C25-Alkylrest stehen, der sich von einer C16-C24-Fettsäuremischung ableitet, die 45 bis 99 Gew.-% C18-C24-Fettsäure enthält.

5. Verwendung von Estern der allgemeinen Formel (I) nach mindestens einem der Ansprüche 1 bis 3, wobei Mischungen von Estern der Formel (I) in Verhältnis verwendet werden, in denen R²=Wasserstoff : R²=C(=O)R^{1'} von 5:95 bis 35:65, vorzugsweise von 10:90 bis 30:70, verwendet werden.

6. Perlglanzkonzentrate für wässrige, tensidische Zubereitungen enthaltend
a) 7,5 bis 35 Gew.-% Ester des 2,5-Di(hydroxymethyl)tetrahydrofurans der allgemeinen Formel (I) gemäß Anspruch 1
b) 0,1 bis 60 Gew% anionische, nichtionische, kationische, ampholytische oder zwitterionische Tenside oder deren Kombinationen
c) 0 - 40 Gew.-% Polyole
d) ad 100 Gew.-% Wasser.

7. Perlglanzkonzentrate nach Anspruch 6, **dadurch gekennzeichnet, dass** sie in Mengen von 15 bis 30 Gew.-% Ester der allgemeinen Formel (I) enthalten wie in einem der Ansprüche 1 bis 5 definiert.

8. Perlglanzkonzentrate nach Anspruch 6 oder 7, **dadurch gekennzeichnet, dass** sie b) anionische Tenside oder nichtionische Tenside oder Kombinationen von nichtionischen und zwitterionischen Tenside enthalten.

9. Perlglanzkonzentrate nach einem der Ansprüche 6 bis 8, **dadurch gekennzeichnet, dass** sie b) 10 bis 30 Gew.-% anionische Tenside oder nichtionische Tenside oder Kombinationen von nichtionischen und zwitterionischen Tenside enthalten.

10. Verfahren zur Herstellung von Perlglanzkonzentraten nach einem der Ansprüche 6 bis 9 **dadurch gekennzeichnet, dass** man die Ester des 2,5-Di(hydroxymethyl)tetrahydrofurans der allgemeinen Formel (I) gemäß Anspruch 1 sowie die Tenside (b) gemäß Anspruch 6 sowie ggf. die Polyole (c) und ad 100 Gew.-% Wasser unter Rühren auf Temperaturen erwärmt, die 5 bis 20 °C über dem Schmelzpunkt des Esters (a) liegen, rührt und anschließend die Mischung unter stetigem Rühren auf etwa Raumtemperatur (20 bis 23 °C) abkühlt.

11. Verfahren zur Herstellung von Perlglanzkonzentraten nach einem der Ansprüche 6 bis 9, **dadurch gekennzeichnet, dass** man zu einer wässrigen Paste der Tenside (b) gemäß Anspruch 6 die Ester des 2,5-Di(hydroxymethyl)tetrahydrofurans der allgemeinen Formel (I) gemäß Anspruch 1 einrührt, auf Temperaturen, die 5 bis 20 °C über dem Schmelzpunkt des Esters liegen, erwärmt, rührt und anschließend mit weiterem Wasser und ggf. Polyolen die gewünschte Konzentration einstellt.

12. Verwendung der Perlglanzkonzentrate nach einem der Ansprüche 6 bis 9 zur Herstellung von wässrigen, tensidischen Zubereitungen mit Perlglanzeffekt.

13. Verwendung der Perlglanzkonzentrate nach Anspruch 12, **dadurch gekennzeichnet, dass** sie in Mengen von 0,2 bis 10 Gew.-% - bezogen auf Zubereitung - verwendet werden.

14. Wässrige, tensidische Zubereitung mit Perlglanzeffekt enthaltend 0,2 bis 10 Gew.-% Perlglanzkonzentrat nach einem der Ansprüche 6 bis 9.

15. Wässrige, tensidische Zubereitung enthaltend 0,015 bis 3,5 Gew.-% Ester des 2,5-Di(hydroxymethyl)tetrahydrofurans der allgemeinen Formel (I) gemäß Anspruch 1.

## Claims

1. The use of esters of 2, 5-di(hydroxymethyl)tetrahydrofuran of the general formula (I), 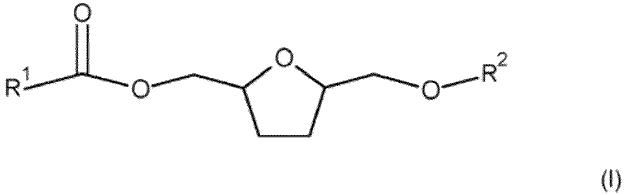 in which
R² is C(=O)R^{1'}, and R¹ and R^{1'} independently of one another are a C13-C25 alkyl radical optionally substituted by a hydroxyl group,
or
R² is hydrogen and R¹ is a C13-C25 alkyl radical optionally substituted by a hydroxyl group, or mixtures of such esters,
as pearlizing wax in aqueous surfactant preparations.

2. The use of esters of the general formula (I) according to claim 1, where at least one of the radicals R¹ and R^{1'} is a linear C13-C25 alkyl radical.

3. The use of esters of the general formula (I) according to claim 1 or 2, where at least one of the radicals R¹ and R^{1'} is a C15-C25 alkyl radical which is derived from a C16-C24 fatty acid mixture having a C18-C24 fatty acid content of 25 to 100 wt%, preferably 35 to 99 wt% and more particularly 45 to 99 wt%, based on fatty acid mixture.

4. The use of esters of the general formula (I) according to any of claims 1 to 3, where R² is C(=O)R^{1'} and the radicals R¹ and R^{1'} are a C17-C25 alkyl radical which derives from a C16-C24 fatty acid mixture which comprises 45 to 99 wt% of C18-C24 fatty acid.

5. The use of esters of the general formula (I) according to at least one of claims 1 to 3, where mixtures of esters of the formula (I) are used in a ratio in which R²=hydrogen:R²=C(=O)R^{1'} is from 5: 95 to 35:65, preferably from 10:90 to 30:70.

6. A pearlizing concentrate for aqueous surfactant preparations, comprising
a) 7.5 to 35 wt% of esters of 2,5-di(hydroxymethyl)-tetrahydrofuran of the general formula (I) according to claim 1
b) 0.1 to 60 wt% of anionic, nonionic, cationic, ampholytic or zwitterionic surfactants or combinations thereof
c) 0-40 wt% of polyols
d) water ad 100 wt%.

7. The pearlizing concentrate according to claim 6, **wherein** it comprises, in amounts of 15 to 30 wt%, esters of the general formula (I) as defined in any of claims 1 to 5.

8. The pearlizing concentrate according to claim 6 or 7, **wherein** it comprises b) anionic surfactants or nonionic surfactants, or combinations of nonionic and zwitterionic surfactants.

9. The pearlizing concentrate according to any of claims 6 to 8, **wherein** it comprises b) 10 to 30 wt% of anionic surfactants or nonionic surfactants, or combinations of nonionic and zwitterionic surfactants.

10. A method for producing a pearlizing concentrate according to any of claims 6 to 9, **wherein** it comprises heating the esters of 2,5-di(hydroxymethyl)tetrahydrofuran of the general formula (I) according to claim 1 and also the surfactants (b) according to claim 6 and also, optionally the polyols (c) and water ad 100 wt%, with stirring, to temperatures 5 to 20°C above the melting point of the ester (a), stirring the mixture and then cooling it, with continual stirring, to approximately room temperature (20 to 23°C).

11. A method for producing a pearlizing concentrate according to any of claims 6 to 9, **wherein** it comprises stirring the esters of 2,5-di(hydroxymethyl)tetrahydrofuran of the general formula (I) according to claim 1 into an aqueous paste of the surfactants (b) according to claim 6, heating the mixture to temperatures 5 to 20°C above the melting point of the ester, stirring the heated mixture, and then establishing the desired concentration with further water and optionally polyols.

12. The use of the pearlizing concentrate according to any of claims 6 to 9 for producing aqueous surfactant preparations with pearlizing effect.

13. The use of the pearlizing concentrate according to claim 12, **wherein** the concentrate is used in amounts of 0.2 to 10 wt%, based on preparation.

14. An aqueous surfactant preparation with pearlizing effect, comprising 0.2 to 10 wt% of pearlizing concentrate according to any of claims 6 to 9.

15. An aqueous surfactant preparation comprising 0.015 to 3.5 wt% of esters of 2,5-di(hydroxymethyl)tetrahydrofuran of the general formula (I) according to claim 1.

## Revendications

1. Utilisation d'esters de 2,5-di(hydroxyméthyl)-tétrahydrofurane de formule générale (I) 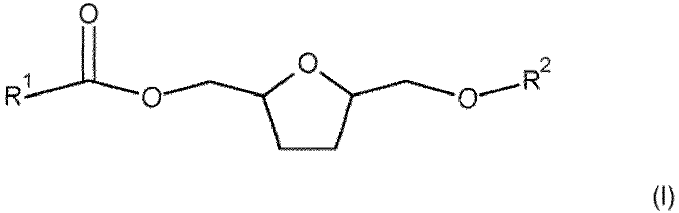 dans laquelle
R² représente C(=O)R^{1'}, et R¹ et R^{1'} représentent indépendamment l'un de l'autre un radical alkyle en C13-C25, qui est éventuellement substitué avec un groupe hydroxyle,
ou
R² représente l'hydrogène et R¹ représente un radical alkyle en C13-C25, qui est éventuellement substitué avec un groupe hydroxyle, ou de mélanges de tels esters,
en tant que cire nacrée dans des préparations tensioactives aqueuses.

2. Utilisation d'esters de formule générale (I) selon la revendication 1, dans laquelle au moins un des radicaux R¹ et R^{1'} représente un radical alkyle en C13-C25 linéaire.

3. Utilisation d'esters de formule générale (I) selon la revendication 1 ou 2, dans laquelle au moins un des radicaux R¹ et R^{1'} représente un radical alkyle en C15-C25, qui est dérivé d'un mélange d'acides gras en C16-C24 ayant une teneur en acide gras en C18-C24 de 25 à 100 % en poids, de préférence de 35 à 99 % en poids, et notamment de 45 à 99 % en poids, par rapport au mélange d'acides gras.

4. Utilisation d'esters de formule générale (I) selon l'une quelconque des revendications 1 à 3, dans laquelle R² représente C(=O)R^{1'}, et les radicaux R¹ et R^{1'} représentent un radical alkyle en C17-C25, qui est dérivé d'un mélange d'acides gras en C16-C24 qui contient 45 à 99 % en poids d'acide gras en C18-C24.

5. Utilisation d'esters de formule générale (I) selon au moins l'une quelconque des revendications 1 à 3, dans laquelle des mélanges d'esters de formule (I) sont utilisés en un rapport R² = hydrogène : R² = C(=O)R^{1'} de 5:95 à 35:65, de préférence de 10:90 à 30:70.

6. Concentrés nacrés pour préparations tensioactives aqueuses, contenant :
a) 7,5 à 35 % en poids d'esters de 2,5-di(hydroxyméthyl)tétrahydrofurane de formule générale (I) selon la revendication 1,
b) 0,1 à 60 % en poids de tensioactifs anioniques, non ioniques, cationiques, ampholytiques ou zwitterioniques ou leurs combinaisons,
c) 0 à 40 % en poids de polyols,
d) jusqu'à 100 % en poids d'eau.

7. Concentrés nacrés selon la revendication 6, **caractérisés en ce qu'**ils contiennent en quantités de 15 à 30 % en poids des esters de formule générale (I) tels que définis dans l'une quelconque des revendications 1 à 5.

8. Concentrés nacrés selon la revendication 6 ou 7, **caractérisés en ce qu'**ils contiennent b) des tensioactifs anioniques ou des tensioactifs non ioniques ou des combinaisons de tensioactifs non ioniques et zwitterioniques.

9. Concentrés nacrés selon l'une quelconque des revendications 6 à 8, **caractérisés en ce qu'**ils contiennent b) 10 à 30 % en poids de tensioactifs anioniques ou de tensioactifs non ioniques ou de combinaisons de tensioactifs non ioniques et zwitterioniques.

10. Procédé de fabrication de concentrés nacrés selon l'une quelconque des revendications 6 à 9, **caractérisé en ce que** les esters de 2,5-di(hydroxyméthyl)-tétrahydrofurane de formule générale (I) selon la revendication 1, ainsi que les tensioactifs (b) selon la revendication 6, ainsi qu'éventuellement les polyols (c) et jusqu'à 100 % en poids d'eau sont portés sous agitation à des températures qui se situent 5 à 20 °C au-dessus du point de fusion de l'ester (a), agités, puis le mélange est refroidi sous agitation constante à environ la température ambiante (20 à 23 °C).

11. Procédé de fabrication de concentrés nacrés selon l'une quelconque des revendications 6 à 9, **caractérisé en ce que** les esters de 2,5-di(hydroxyméthyl)-tétrahydrofurane de formule générale (I) selon la revendication 1 sont incorporés dans une pâte aqueuse des tensioactifs (b) selon la revendication 6, portés à des températures qui se situent 5 à 20 °C au-dessus du point de fusion de l'ester, agités, puis la concentration souhaitée est ajustée avec davantage d'eau et éventuellement des polyols.

12. Utilisation des concentrés nacrés selon l'une quelconque des revendications 6 à 9 pour la fabrication de préparations tensioactives aqueuses à effet nacré.

13. Utilisation des concentrés nacrés selon la revendication 12, **caractérisée en ce qu'**ils sont utilisés en quantités de 0,2 à 10 % en poids, par rapport à la préparation.

14. Préparation tensioactive aqueuse à effet nacré, contenant 0,2 à 10 % en poids de concentré nacré selon l'une quelconque des revendications 6 à 9.

15. Préparation tensioactive aqueuse contenant 0,015 à 3,5 % en poids d'esters de 2,5-di(hydroxyméthyl)-tétrahydrofurane de formule générale (I) selon la revendication 1.
